# FASCICULE DE BREVET EUROPEEN

(11) **EP 4 514 231 B1**
(45) Date de publication et mention de la délivrance du brevet: **11.11.2026**
(21) Numéro de dépôt: 23722319.3
(22) Date de dépôt: 25.04.2023
(51) Int. Cl.: A61B 17/02, A61B 17/08, A61B 17/00, A61B 90/00, A61B 17/32

(54) **DISPOSITIF DE TRACTION ENDOSCOPIQUE AJUSTABLE**
EINSTELLBARE ENDOSKOPISCHE ZUGVORRICHTUNG
ADJUSTABLE ENDOSCOPIC TRACTION DEVICE

(30) Priorité: 26.04.2022 FR 2203841
(43) Date de publication de la demande: 05.03.2025
(73) Titulaire: Hospices Civils de Lyon, 69229 Lyon Cedex 02 (FR); Université Claude Bernard Lyon 1, 69100 Villeurbanne (FR); A-Tract Device & Co, 69380 Belmont d'Azergues (FR)
(72) Inventeur: MASGNAUX, Louis-Jean, 69002 Lyon (FR); GRIMALDI, Jean, 69002 Lyon (FR); BRUN, Soline, 69008 Lyon (FR); PIOCHE, Mathieu, 69380 Belmont d'Azergues (FR); RIVORY, Jérôme, 69390 Vourles (FR)
(74) Mandataire: Opilex
(86) Numéro de dépôt international: PCT/EP2023/060803
(87) Numéro de publication internationale: WO 2023/208930

(56) Documents cités:
- WO-A1-2020/018566
- WO-A1-2021/009835
- WO-A1-2022/015931
- WO-A2-2010/042844
- US-A1- 2021 169 464

## Description

### Domaine technique de l'invention

La présente invention se rapporte au domaine technique des dispositifs endoscopiques et plus particulièrement les dispositifs de traction d'un tissu biologique par exemple utilisés dans la résection de tumeurs superficielles du tube digestif.

### État de la technique

On connait les dispositifs de traction utilisés dans la résection de tumeurs superficielles du tube digestif. Un tel dispositif de traction comprenant généralement une ancre proximale et un organe élastique configuré pour exercer un effort de traction proximale sur l'ancre proximale.

En pratique, la technique employée pour réséquer une tumeur superficielle du tube digestif, est la dissection sous-muqueuse. Cette dernière est une technique complexe permettant de réséquer de manière mini-invasive des tumeurs superficielles. La dissection sous-muqueuse consiste à séparer la muqueuse tumorale du reste de la paroi digestive, et notamment du muscle, en se servant de la sous-muqueuse comme plan de dissection. Cette intervention est dite mini invasive, car elle respecte l'organe avec des temps de cicatrisation relativement courts par rapport aux interventions invasives traditionnelles.

Un dispositif de traction permet de faciliter la dissection sous-muqueuse en éloignant, par traction, la muqueuse du muscle de manière à exposer la sous-muqueuse. Pour ce faire, l'ancre proximale est fixée sur une partie proximale de la muqueuse tumorale au moyen d'un clip endoscopique hémostatique et l'organe élastique est fixé sur la paroi opposée du tube digestif de sorte à exercer sur la partie proximale de la muqueuse tumorale un effort de traction permettant de séparer ladite muqueuse tumorale du reste de la paroi digestive.

Néanmoins, les dispositifs de traction actuels ne sont pas entièrement satisfaisants. En effet, après avoir découpé la première partie de la sous-muqueuse, sous la partie proximale de la muqueuse tumorale, ladite partie proximale se détend et l'effort de traction exercé par l'organe élastique sur l'ancre proximale devient insuffisant pour exposer convenablement la sous-muqueuse. Il est alors difficile d'insérer l'outil de dissection et de poursuivre l'opération de dissection dans de bonnes conditions.

Face à cet état de fait, l'invention a pour objectif de fournir un dispositif de traction permettant d'exposer de manière suffisante la sous-muqueuse de sorte à permettre le passage de l'outil de dissection dans de bonnes conditions pendant toute la durée de l'opération de résection de la muqueuse tumorale.

Le document WO2010042844 décrit un dispositif de traction pour les chirurgies internes. Le dispositif comprend des pinces solidaires des angles d'un filet élastique rectangulaire. La distance entre une des pinces et le filet élastique est réglable par l'intermédiaire d'une chaine de billes coopérant avec un bloc anti-retour.

Le document US2021169464 décrit un dispositif de traction muni d'une ancre proximale, d'une ancre distale et d'un élément élastique exerçant une traction sur les ancres distale et proximale lorsqu'elles sont écartées.

### Divulgation de l'invention

La solution proposée par l'invention est un dispositif de traction d'un tissu biologique tel que défini dans la revendication 1 annexée. L'invention porte également sur les variantes des revendications dépendantes.

### Description des figures

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
[Fig.1] est une vue schématique d'un premier exemple de réalisation du dispositif de traction objet de l'invention ;
[Fig.2] est une vue schématique d'un deuxième exemple de réalisation du dispositif de traction objet de l'invention ;
[Fig.3] est une vue schématique d'un troisième exemple de réalisation du dispositif de traction objet de l'invention ;
[Fig.4] est une vue schématique de l'exemple de réalisation du dispositif de traction de la figure [Fig.1] agencé au niveau d'un tissu biologique à réséquer au sein d'un tube digestif ;
[Fig.5] est une vue schématique de l'exemple de réalisation du dispositif de traction de la figure [Fig.1] après fixation des ancres sur le tissu biologique et fixation de l'organe élastique sur le tube digestif en vis-à-vis du tissu biologique et avant dissection de la partie proximale de la sous-muqueuse au moyen de l'outil de dissection endoscopique ;
[Fig.6] est une vue schématique de l'exemple de réalisation du dispositif de traction de la figure [Fig.1] après dissection de la partie proximale de la sous-muqueuse exposée à la [Fig.5] ;
[Fig.7] est une vue schématique de l'exemple de réalisation du dispositif de traction de la figure [Fig.1] après réglage de la longueur proximale par traction sur l'extrémité du corps du collier de serrage et avant dissection de la sous-muqueuse nouvellement exposée ;
[Fig.8] est une vue schématique de l'exemple de réalisation du dispositif de traction de la figure [Fig.1] après dissection de la partie proximale de la sous-muqueuse exposée à la [Fig.7] après réglage de la longueur proximale par traction sur l'extrémité du corps du collier de serrage et avant dissection du reste de la sous-muqueuse,
[Fig.9] est une vue schématique de l'exemple de réalisation du dispositif de traction de la figure [Fig.1] à l'intérieur du dispositif de largage, avant largage sur la zone d'opération ;
[Fig.10] est une vue schématique de l'exemple de réalisation du dispositif de traction de la figure [Fig.1] à l'extérieur du dispositif de largage, après largage sur la zone d'opération.

### Description détaillée

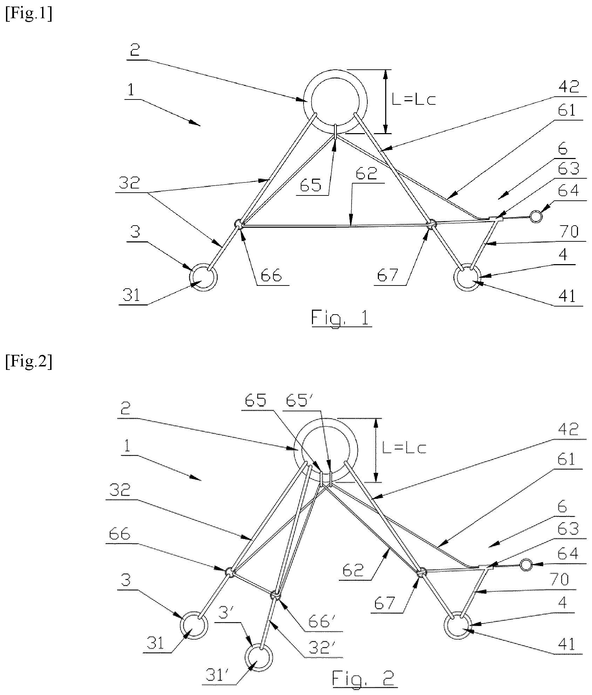

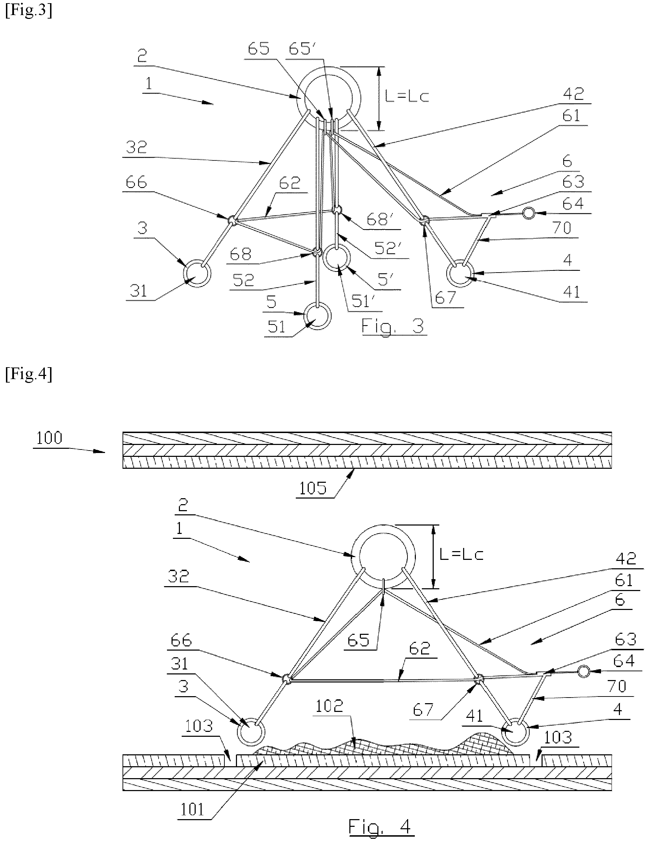

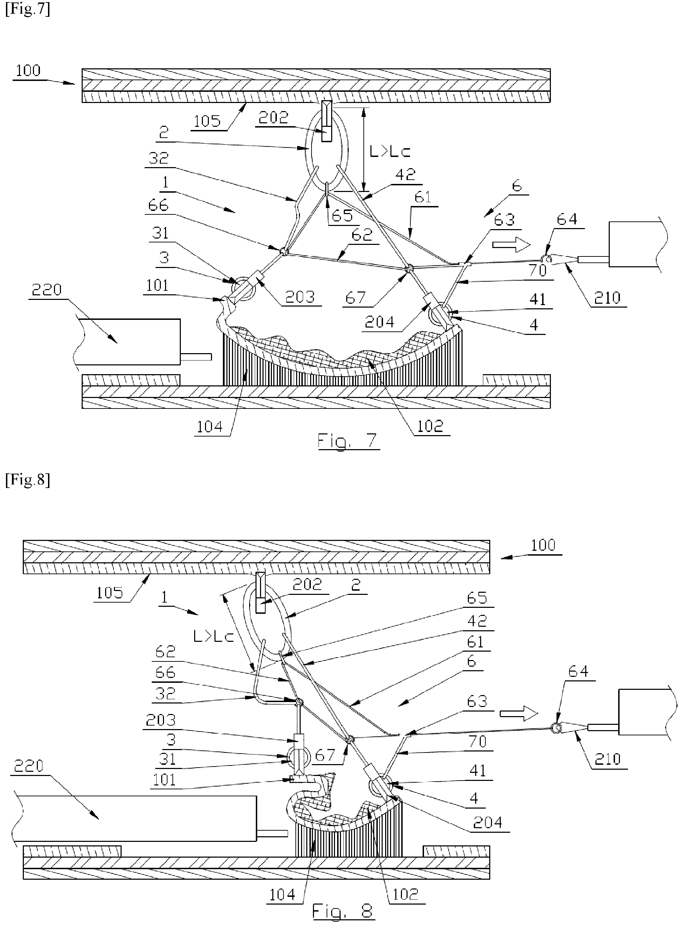

En se rapportant aux figures [Fig.1] à [Fig.8], l'invention concerne un dispositif de traction (1) d'un tissu biologique (101). Dans la pratique, le tissu biologique (101) peut prendre la forme d'une muqueuse tumorale comme représentée sur les figures [Fig.4] à [Fig.8]. Le tissu biologique est généralement agencé dans une cavité du corps humain tel que le tube digestif.

Le dispositif de traction (1) comprend une ancre proximale (3). Cette dernière peut se présenter sous la forme d'une boucle de forme circulaire comme dans l'exemple de réalisation de la figure 1, de forme triangulaire, de forme rectangulaire, ou de toute autre forme convenant à l'homme du métier. Cette boucle peut être rigide, flexible, ou élastique. Par exemple, la boucle peut être réalisée en métal, en matière plastique, en latex, ou tout autre matériau convenant à l'homme du métier. La boucle peut également être réalisée au moyen d'un fil en coton, en soie, en polyester, en nylon, ou tout autre matériau convenant à l'homme du métier. Préférentiellement, l'ancre proximale (3) est réalisée dans un ou plusieurs matériaux hypoallergéniques du type polyglécaprone, polybutester, glycomère, poly-glactin, polyglyconate, polydioxanone, polypropylène. Astucieusement, l'ancre proximale (3) comporte un revêtement de surface réalisée dans un ou plusieurs matériaux hypoallergéniques du type de ceux précédemment décrits. Préférentiellement, la boucle formant l'ancre proximale (3) présente un orifice (31) configuré pour permettre le passage d'un mors d'un clip endoscopique hémostatique (203). En particulier, la boucle peut présenter un orifice (31) s'étendant au-delà d'un demi-cercle de 8mm de diamètre. De manière générale, un clip endoscopique hémostatique se présente sous la forme d'une pince comportant deux mors striés. Le clip endoscopique hémostatique est configuré pour se fixer sur un tissu biologique par pincement dudit tissu biologique entre ses mors.

Le dispositif de traction (1) comprend également une ancre distale (4). Cette dernière peut se présenter sous la forme d'une boucle de forme circulaire comme dans l'exemple de réalisation de la figure 1, de forme triangulaire, de forme rectangulaire, ou de toute autre forme convenant à l'homme du métier. Cette boucle peut être rigide, flexible, ou élastique. Par exemple, la boucle peut être réalisée en métal, en matière plastique, en latex, ou tout autre matériau convenant à l'homme du métier. La boucle peut également être réalisée au moyen d'un fil en coton, en polyester, en nylon, ou tout autre matériau convenant à l'homme du métier. Préférentiellement, l'ancre distale (4) est réalisée dans un ou plusieurs matériaux hypoallergéniques du type de ceux précédemment décrits. Astucieusement, l'ancre distale (4) comporte un revêtement de surface réalisée dans un ou plusieurs matériaux hypoallergéniques de ceux précédemment décrits. Préférentiellement, la boucle formant l'ancre distale (4) présente un orifice (41) configuré pour permettre le passage d'un mors d'un clip endoscopique hémostatique (204). En particulier, la boucle peut présenter un orifice (41) s'étendant au-delà d'un demi-cercle de 5 mm de diamètre.

Le dispositif de traction (1) comprend encore un organe élastique (2). Ce dernier comporte au moins une partie extensible de longueur (L) et de module de Young (E). Par « au moins une partie extensible », on entend que l'organe élastique (2) peut être entièrement élastique, ou peut comporter une ou plusieurs parties élastiques. Dans les exemples de réalisation représentés sur les figures [Fig.1] à [Fig. 8], la partie extensible peut être réalisée en matière élastique de module de Young (E). En pratique, la partie extensible peut être réalisée en élastomère, par exemple en caoutchouc, en élastomère thermoplastique (TPE), en polyéthylène téréphtalate (PET), etc. Astucieusement, la partie extensible est réalisée en élastomère à base de latex. Préférentiellement, la partie extensible est réalisée dans un matériau hypoallergénique du type élastomère à base de silicone. Lorsque le matériau utilisé pour la partie extensible présente des risques allergiques, la partie extensible peut présenter un revêtement en matière hypoallergénique de module de Young (Er) inférieur ou égale au module de Young (E) et de type élastomère à base de silicone. La partie extensible peut avoir une forme d'anneau comme dans les exemples de réalisation des figures [Fig.1] à [Fig.3]. L'anneau peut avoir un diamètre compris entre 3 mm et 10 mm et un diamètre de section compris entre 0.5 mm et 3 mm. Dans des variantes de réalisation, la partie élastique peut prendre la forme d'une bande, d'un cylindre, ou toute autre forme convenant à l'homme du métier. Dans d'autres variantes de réalisation, la partie extensible peut se présenter sous la forme d'un ressort.

Le module de Young (E) est inférieur à 0.1 GPa. Avantageusement, le module de Young (E) est compris entre 0.001 GPa et 0.05 GPa. Préférentiellement, le module de Young (E) est compris entre 0.008 GPa et 0.03 GPa.

L'organe élastique (2) peut présenter un orifice configuré pour permettre le passage d'un mors d'un clip endoscopique hémostatique (202). Ce passage peut se présenter sous la forme d'un orifice aménagé sur l'organe élastique (2). Dans des variantes de réalisation, le passage peut se présenter sous la forme d'une pièce rapportée cylindrique, torique ou de toute autre forme convenant à l'homme du métier. En particulier, le passage s'étendant au-delà d'un demi-cercle de 5 mm de diamètre. Lorsque la partie extensible de l'organe élastique (2) a une forme d'anneau, l'orifice de l'anneau peut jouer le rôle de passage.

La partie extensible est configurée pour exercer un effort de traction sur l'ancre distale (4) et sur l'ancre proximale (3) lorsque la longueur (L) est étendue au-delà d'une longueur contractée (Lc).

L'ancre proximale (3) est montée mobile par rapport à l'organe élastique (2) entre une distance proximale minimale et une distance proximale maximale. La distance proximale minimale est préférentiellement comprise entre 0 mm et 10 mm. La distance proximale maximale est préférentiellement comprise entre 10 mm et 50 mm. Comme dans l'exemple de réalisation représenté sur la figure [Fig.1], l'ancre proximale (3) peut être montée mobile par rapport à l'organe élastique (2) par l'intermédiaire d'un fil proximal (32). Ce dernier peut être réalisé en coton, en polyester, en nylon, ou tout autre matériau convenant à l'homme du métier. Le fil proximal peut avoir une longueur comprise entre 10 mm et 50 mm. Dans des variantes de réalisation, l'ancre proximale (3) peut être montée mobile par rapport à l'organe élastique (2) par l'intermédiaire d'une bande flexible, ou d'une tige flexible.

Le dispositif traction (1) comprend un organe de réglage (6). Ce dernier est configuré pour régler la distance proximale maximale entre une distance proximale maximale initiale et une distance proximale maximale finale inférieure à la distance proximale maximale initiale.

Dans un premier mode de réalisation de l'organe de réglage représenté sur la figure [Fig.1], l'organe de réglage (6) peut comprendre un collier de serrage (61). Ce dernier comporte un corps (62) et une cage (63). Le corps (62) peut avoir un profil de forme cylindrique, parallélépipédique ou toute autre forme de profil convenant à l'homme du métier. Le corps (62) à un diamètre, ou une épaisseur, préférentiellement compris entre 0.2 mm et 5 mm. Le corps (62) peut avoir une longueur comprise entre 5 cm et 20 cm. La cage (63) est configurée pour permettre le passage du corps (62) de manière à former une courbe fermée d'une circonférence de longueur variable. Le corps (62) peut comporter un ou plusieurs repères visuels, chacun desdits repères visuels permettant d'indiquer une longueur de circonférence donnée lorsque ledit repère visuel est agencé au niveau de la cage (63). En particulier, les repères visuels peuvent prendre la forme de traits de couleurs réparties le long du corps (62). Dans une variante de réalisation, les repères visuels peuvent prendre la forme de zones de couleurs réparties le long du corps (62). Préférentiellement, le collier de serrage (61) comprend entre 2 et 6 repères visuels. L'extrémité du corps (62), opposé à la cage (63), peut comporter une boucle de tirage (64). Cette boucle de tirage (64) est préférentiellement configurée pour permettre la préhension par une pince chirurgicale endoscopique (210). En particulier, la boucle de tirage (64) peut avoir un diamètre compris entre 5mm et 20mm. Le collier de serrage (61) peut être réalisé dans matériau plastique, composite ou tout autre matériau convenant à l'homme du métier. Préférentiellement, le collier de serrage (61) peut être réalisé dans un matériau hypoallergénique du type de ceux précédemment décrits. Le collier de serrage (61) peut astucieusement être réalisé dans un matériau à mémoire de forme de type nitinol.

Le collier de serrage (61) peut comporter un mécanisme anti-retour configuré pour empêcher le corps (62) de coulisser dans un sens dans lequel sa longueur de circonférence augmente. Le mécanisme anti-retour peut se présenter sous toute forme convenant à l'homme du métier.

En particulier, le mécanisme antiretour peut se présenter sous la forme de poils anti-retours, ou de lamelles anti-retours, souples agencés sur le corps (62) du collier de serrage (61) de manière à :
- se rapprocher du corps (62) lorsque la partie dudit corps sur laquelle sont fixés lesdits poils anti-retours, ou lesdites lamelles anti-retours, se déplace à l'intérieur de la cage (63) dans le sens réduisant la longueur de la circonférence,
- s'éloigner du corps (62) et entrer en butée avec la cage (63) lorsque la partie dudit corps sur laquelle sont fixés lesdits poils anti-retours, ou lesdites lamelles anti-retours, tend à se déplacer à l'intérieur de ladite cage dans le sens augmentant la longueur de la circonférence.

Dans une variante de réalisation, le mécanisme anti-retour peut se présenter sous la forme de butées agencées le long du corps (62) et d'une ou plusieurs lamelles souples agencées à l'intérieure de la cage (63), ladite ou lesdites lamelles étant configurées pour :
- s'éloigner du corps (62) de manière à laisser passer les butées lorsque ledit corps se déplace à l'intérieur de la cage (63) dans le sens réduisant la longueur de la circonférence,
- se rapprocher du corps (62) de manière à faire obstacle aux butées lorsque ledit corps tend à se déplacer à l'intérieur de la cage (63) dans le sens réduisant la longueur de la circonférence.

Dans d'autres exemples de réalisation, le mécanisme anti-retour comporte des pièces crantées agencées mobiles à l'intérieur d'un cône formé dans la cage (63) de sorte que les pièces crantées :
- s'éloignent les unes des autres de manière à laisser passer le corps (62) lorsque ce dernier se déplace à l'intérieur de la cage (63) dans le sens réduisant la longueur de la circonférence,
- se rapprocher les unes des autres de manière à exercer une pression sur le corps (62) et faire obstacle à l'avance dudit corps à l'intérieur la cage (63) lorsque ledit corps tend à se déplacer à l'intérieur de ladite cage dans le sens réduisant la longueur de la circonférence.

En se rapportant aux exemples de réalisation représentés sur les figures [Fig.1 à Fig.3], l'organe de réglage (6) peut également comprendre un guide central (65) agencé sur l'organe élastique (2). Le guide central (65) est configuré pour permettre le passage du corps (62) du collier de serrage (61). Bien que certains modes de réalisation ne comportent pas de guide central, la présence d'un guide central (65) améliore la fonction de réglage de l'organe de réglage (6).

Comme représenté sur les figures [Fig.1 à Fig.3], le guide central (65) peut se présenter sous la forme d'un anneau monté sur l'organe élastique (2). Le guide central (65) peut également se présenter sous la forme d'un orifice aménagé sur l'organe élastique (2). Dans des variantes de réalisation, le guide central (65) peut se présenter sous la forme d'une pièce rapportée cylindrique, torique ou de toute autre forme convenant à l'homme du métier. Le guide central (65) peut présenter un passage dont les dimensions sont au moins supérieures de 0.2mm, préférentiellement supérieures de 0.5mm, au diamètre, ou à l'épaisseur, du corps (62) du collier de serrage (61).

L'organe de réglage (6) peut encore comprendre un guide proximal (66) configuré pour permettre le passage du corps (62) du collier de serrage (61). Le guide proximal (66) peut être agencé sur l'ancre proximale (3). Le guide proximal (66) peut prendre toute forme convenant à l'homme du métier.

En particulier, lorsque l'ancre proximale (3) est réalisée au moyen d'un fil, le guide proximal (66) peut se présenter sous la forme d'une pièce rapportée cylindrique, torique ou de toute autre forme convenant à l'homme du métier.

Également, lorsque l'ancre proximale (3) est réalisée dans un matériau rigide, le guide proximal (66) peut être formé d'une seule pièce avec le corps de ladite ancre proximale. Le guide proximal (66) peut alors avoir la forme d'un orifice agencé sur le corps de l'ancre proximale (3) ou encore sous la forme d'un cylindre, d'un anneau, ou autre, agencé en saillit sur le corps de l'ancre proximale (3) vers l'intérieur ou vers l'extérieur de ladite ancre proximale.

Dans un exemple particulier de réalisation, l'orifice (31) de l'ancre proximale (3) fait office de guide proximal (66).

Le guide proximal (66) peut alternativement être agencé sur le fil proximal (32). Le guide proximal (66) peut notamment être agencé à l'extrémité du fil proximal (32). Le guide proximal (66) peut encore être agencé entre deux parties du fil proximal (32) comme dans les exemples de réalisation représentés sur les figures [Fig.1] à [Fig.8]. Le guide proximal (66) est avantageusement agencé à proximité de l'ancre proximale (3). Le guide proximal (66) peut se présenter sous la forme d'une pièce rapportée cylindrique, torique ou de toute autre forme convenant à l'homme du métier. Lorsque l'ancre proximale (3) est une bande, le guide proximal (66) peut se présenter sous la forme d'un œillet.

Le guide proximal (66) peut présenter un passage dont les dimensions sont au moins supérieures de 0.2mm, préférentiellement supérieures de 0.5mm, au diamètre, ou à l'épaisseur, du corps (62) du collier de serrage (61).

Dans ce premier mode de réalisation de l'organe de réglage (6), le collier de serrage (61) est alors configuré pour diminuer la distance proximale maximale lorsque la longueur de sa circonférence diminue.

Dans un deuxième mode de réalisation non représenté de l'organe de réglage (6), ce dernier peut comprendre une bobine proximale configurée pour enrouler le fil proximal (32) lorsque l'effort de traction exercé sur ledit fil proximal est inférieur à un effort de rappel. L'effort de rappel est préférentiellement compris entre 0.2 N et 1.8 N. Dans un exemple de réalisation, la bobine proximale comprend un tambour configuré pour recevoir les enroulements du fil proximal (32). La bobine proximale comprend également un carter sur lequel est monté mobile en rotation le tambour. La bobine proximale comprend enfin un ressort de rappel configuré pour déclencher l'enroulement du fil proximal (32) autour du tambour lorsque l'effort de traction exercé sur ledit fil proximal est inférieur à l'effort de rappel. Le carter peut être fixé sur l'organe élastique (2). Astucieusement, dans une variante de réalisation, le ressort de rappel fait office d'organe élastique (2).

L'ancre distale (4) peut être montée mobile par rapport à l'organe élastique (2) entre une distance distale minimale et une distance distale maximale. La distance distale minimale est préférentiellement comprise entre 0 mm et 10 mm. La distance distale maximale est préférentiellement comprise entre 10 mm et 50 mm. Dans un mode préféré de réalisation de l'ancre distale (4), la distance distale maximale est inférieure ou égale à 1/3 de la distance proximale maximale. Comme dans l'exemple de réalisation représenté sur les figures [Fig.1] à [Fig. 8], l'ancre distale (4) peut être montée mobile par rapport à l'organe élastique (2) par l'intermédiaire d'un fil distal (42). Ce dernier peut être réalisé en coton, en polyester, en nylon, ou tout autre matériau convenant à l'homme du métier. Le fil distal (42) peut avoir une longueur comprise entre 10 mm et 50 mm. Dans des variantes de réalisation, l'ancre distale (4) peut être montée mobile par rapport à l'organe élastique (2) par l'intermédiaire d'une bande flexible, ou d'une tige flexible.

L'organe de réglage (6) peut alors être configuré pour régler la distance distale maximale entre une distance distale maximale initiale et une distance distale maximale finale inférieure à la distance distale maximale initiale.

Selon le premier mode de réalisation de l'organe de réglage (6), ce dernier peut comprendre un guide distal (67) configuré pour permettre le passage du corps (62) du collier de serrage (61). Le guide distal (67) peut être agencé sur l'ancre distale (4).

Le guide distal (67) peut prendre toute forme convenant à l'homme du métier.

En particulier, lorsque l'ancre distale (4) est réalisée au moyen d'un fil, le guide distal (67) peut se présenter sous la forme d'une pièce rapportée cylindrique, torique ou de toute autre forme convenant à l'homme du métier.

Également, lorsque l'ancre distale (4) est réalisée dans un matériau rigide, le guide distal (67) peut être formé d'une seule pièce avec le corps de ladite ancre distal. Le guide distal (67) peut alors avoir la forme d'un orifice agencé sur le corps de l'ancre distale (4) ou encore sous la forme d'un cylindre, d'un anneau, ou autre, agencé en saillit sur le corps de l'ancre distale (4) vers l'intérieur ou vers l'extérieur de ladite ancre distale.

Dans un exemple particulier de réalisation, l'orifice (41) de l'ancre distale (4) fait office de guide distal (67).

Le guide distal (67) peut alternativement être agencé sur le fil distal (42). Le guide distal (67) peut notamment être agencé à l'extrémité du fil distal (42). Le guide distal (67) peut encore être agencé entre deux parties du fil distal (42) comme dans les exemples de réalisation représentés sur les figures [Fig.1] à [Fig.8]. Dans ce cas, le guide distal (67) est avantageusement agencé à proximité de l'ancre distale (4). Le guide distal (67) peut se présenter sous la forme d'une pièce rapportée cylindrique, torique ou de toute autre forme convenant à l'homme du métier. Lorsque l'ancre distale (4) est une bande, le guide distal (67) peut se présenter sous la forme d'un œillet.

Le guide distal (67) peut présenter un passage dont les dimensions sont au moins supérieures de 0.2mm, préférentiellement supérieures de 0.5mm, au diamètre, ou à l'épaisseur, du corps (62) du collier de serrage (61).

Dans ce premier mode de réalisation de l'organe de réglage (6), le collier de serrage (61) peut être configuré pour diminuer la distance distale maximale lorsque la longueur de sa circonférence diminue.

Dans un deuxième mode de réalisation non représenté de l'organe de réglage (6), ce dernier peut comprendre une bobine distale configurée pour enrouler le fil distal (42) lorsque l'effort de traction exercé sur ledit fil distal est inférieur à un effort de rappel. L'effort de rappel est préférentiellement compris entre 0.2 N et 1.8 N. Dans un exemple de réalisation, la bobine distale comprend un tambour configuré pour recevoir les enroulements du fil distal (42). La bobine distale comprend également un carter sur lequel est monté mobile en rotation le tambour. La bobine distale comprend enfin un ressort de rappel configuré pour déclencher l'enroulement du fil distal (42) autour du tambour lorsque l'effort de traction exercé sur ledit fil distal est inférieur à l'effort de rappel. Le carter peut être fixé sur l'organe élastique (2). Astucieusement, dans une variante de réalisation, le ressort de rappel fait office d'organe élastique (2). L'organe élastique (2) comprend alors plusieurs éléments élastiques sous la forme d'un ressort de rappel de la bobine proximale et d'un ressort de rappel de la bobine distale. Astucieusement, le carter de la bobine proximal et le carter de la bobine distal forment un unique carter.

L'ancre proximale (3) peut être montée mobile par rapport à l'ancre distale (4) entre une distance d'écartement minimale et une distance d'écartement maximale. La distance d'écartement minimale est préférentiellement comprise entre 0 mm et 10 mm. La distance d'écartement maximale est préférentiellement comprise entre 10 mm et 50 mm. En pratique, l'ensemble des modes de réalisations précédemment décrits pour l'ancre distale (4) et l'ancre proximale (3) permettent une mobilité de l'ancre proximale (3) par rapport à l'ancre distale (4).

L'organe de réglage (6) peut alors être configuré pour réduire la distance d'écartement maximale entre une distance d'écartement maximale initiale et une distance d'écartement maximale finale inférieure à la distance d'écartement maximale initiale. En pratique, l'ensemble des modes de réalisations précédemment décrits pour l'organe de réglage (6) permet de réduire la distance d'écartement maximale.

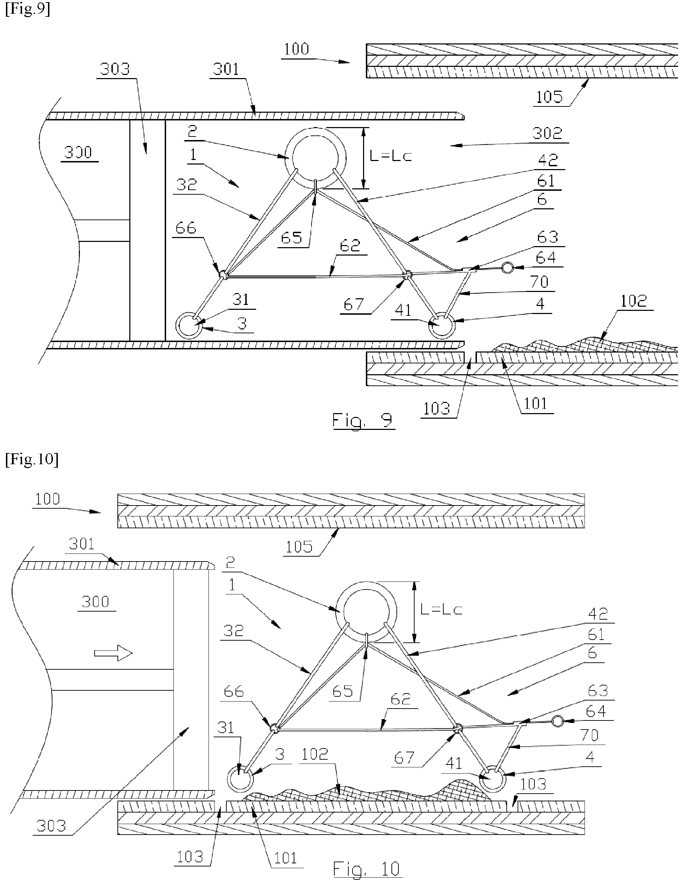

Comme représenté sur les figures [Fig.1] à [Fig.10], l'organe de réglage (6) peut comprendre un lien de serrage (70) configuré pour maintenir la cage (63) à une distance de serrage déterminée de l'ancre distale (4). La distance de serrage est préférablement comprise entre 0 mm et 20 mm. La distance de serrage est préférentiellement inférieure à 5 mm. Ce lien de serrage (70) peut se présenter sous la forme d'un fil s'étendant de la cage (63) du collier de serrage (61) vers l'ancre distale (4). Le fil peut être réalisé en coton, en polyester, en nylon, ou tout autre matériau convenant à l'homme du métier. Alternativement, le lien de serrage (70) peut se présenter sous la forme par une bande, une tige, une bague ou tout autre lien mécanique convenant à l'homme du métier. Le lien de serrage (70) peut alors être réalisé en tissu, en matière plastique, en métal, ou tout matériau convenant à l'homme du métier. Préférentiellement, le matériau du lien de serrage (70) est hypoallergénique, ou lorsqu'il ne l'est pas, ledit lien de serrage (70) comporte un revêtement hypoallergénique.

Dans une variante de réalisation au lien de serrage, l'organe de réglage (6) peut encore comprendre un guide de serrage configuré pour permettre le passage du corps (62) du collier de serrage (61) de sorte que la cage (63) dudit collier de serrage soit confinée entre le guide distal (67) et ledit guide de serrage.

Le guide de serrage peut être agencé sur l'ancre distale (4). Le guide de serrage peut prendre toute forme convenant à l'homme du métier.

En particulier, lorsque l'ancre distale (4) est réalisée au moyen d'un fil, le guide de serrage peut se présenter sous la forme d'une pièce rapportée cylindrique, torique ou de toute autre forme convenant à l'homme du métier.

Également, lorsque l'ancre distale (4) est réalisée dans un matériau rigide, le guide de serrage peut être formé d'une seule pièce avec le corps de ladite ancre distale. Le guide de serrage peut alors avoir la forme d'un orifice agencé sur le corps de l'ancre distale (4) ou encore sous la forme d'un cylindre, d'un anneau, ou autre, agencé en saillit sur le corps de l'ancre distale (4) vers l'intérieur ou vers l'extérieur de ladite ancre distale.

Le guide de serrage peut alternativement être agencé sur le fil distal (42). Dans ce cas, le guide de serrage est avantageusement agencé à proximité de l'ancre distale (4). Le guide de serrage peut se présenter sous la forme d'une pièce rapportée cylindrique, torique ou de toute autre forme convenant à l'homme du métier. Lorsque l'ancre distale (4) est une bande, le guide de serrage peut se présenter sous la forme d'un œillet.

Le guide de serrage peut encore être agencé sur un fil de serrage. Ce dernier est fixé sur l'ancre distale (4). Le fil de serrage peut être réalisé en coton, en polyester, en nylon, ou tout autre matériau convenant à l'homme du métier. Le fil de serrage peut avoir une longueur comprise entre 5 mm et 20 mm. Dans des variantes de réalisation, le fil de serrage est remplacé par une bande flexible, ou une tige flexible. Le guide de serrage est avantageusement agencé à proximité de l'ancre distale (4). Le guide de serrage peut se présenter sous la forme d'une pièce rapportée cylindrique, torique ou de toute autre forme convenant à l'homme du métier.

Le guide de serrage peut présenter un passage dont les dimensions sont au moins supérieures de 0.2mm, préférentiellement supérieures de 0.5mm, au diamètre, ou à l'épaisseur, du corps (62) du collier de serrage (61).

Comme dans l'exemple de réalisation représenté sur la figure [Fig.2], le dispositif de traction (1) peut comprendre une seconde ancre proximale (3') du même type que l'ancre proximale (3) précédemment décrite. C'est-à-dire que la seconde ancre proximale (3') procède les mêmes caractéristiques que l'ancre proximale (3) précédemment décrite, notamment en termes de forme, de matériau, de mobilité, de distanciation par rapport aux autres éléments, etc. En particulier la seconde ancre proximale (3') peut comprendre un orifice (31') et être montée mobile par rapport à l'organe élastique au moyen d'un second fil proximal (32'). De même que précédemment décrit, la partie extensible de l'organe élastique (2) est configurée pour exercer un effort de traction la seconde ancre proximale (3') lorsque sa longueur (L) est étendue au-delà de la longueur contractée (Lc). En outre, la seconde ancre proximale (3') peut être montée mobile par rapport à l'ancre proximale (3) précédemment décrite entre une distance frontale minimale et une distance frontale maximale.

De même, l'organe de réglage (6) peut être configuré pour régler la seconde distance proximale maximale entre une seconde distance proximale maximale initiale et une seconde distance proximale maximale finale inférieure à la seconde distance proximale maximale initiale. À ce titre, l'organe de réglage (6) peut comporter un second guide proximal (66') ou une seconde bobine proximale selon le mode de réalisation choisi. L'organe de réglage (6) peut également être configuré pour régler la distance frontale maximale entre une distance frontale maximale initiale et une distance frontale maximale finale inférieure à la distance frontale maximale initiale.

En se rapportant à l'exemple de réalisation représenté sur la figure [Fig.2], l'organe de réglage (6) peut également comprendre un second guide central (65') agencé sur l'organe élastique (2). Le second guide central (65') est du même type que le guide central (65). C'est-à-dire que le second guide central (65') procède les mêmes caractéristiques que le guide central (65) précédemment décrit, notamment en termes de forme, de matériau, d'agencement par rapport l'organe élastique (2), etc. À ce titre, le second guide central (65') est configuré pour permettre le passage du corps (62) du collier de serrage (61). Avantageusement, le corps (62) du collier de serrage (61) passe par le guide distal (67), puis par le guide central (65), puis par le guide proximal (66), puis par le second guide proximal (66'), puis par le second guide central (65'). Alternativement, et comme représenté sur la figure [Fig.2], le corps (62) du collier de serrage (61) passe par le guide distal (67), puis par le second guide central (65'), puis le second guide proximal (66'), puis par le guide proximal (66), puis par le guide central (65).

Comme dans l'exemple de réalisation représenté sur la figure [Fig.3], le dispositif de traction (1) peut en outre comprendre une ou plusieurs ancres latérales (5, 5'). Préférentiellement, le dispositif de traction (1) comprend un nombre pair d'ancres latérales (5, 5'). Pour chaque pair d'ancres latérales (5, 5'), l'une des ancres latérales (5, 5') est agencée d'un côté du dispositif de traction (1), l'autre desdites ancres latérales est agencée de l'autre côté dudit dispositif de traction.

Une ancre latérale (5, 5') peut se présenter sous la forme d'une boucle de forme circulaire, de forme triangulaire, de forme rectangulaire ou de toute autre forme convenant à l'homme du métier. Cette boucle peut être rigide, flexible, ou élastique. Par exemple, la boucle peut être réalisée en métal, en matière plastique, en latex, ou tout autre matériau convenant à l'homme du métier. La boucle peut également être réalisée au moyen d'un fil en coton, en polyester en nylon, ou tout autre matériau convenant à l'homme du métier. Préférentiellement, l'ancre latérale (5, 5') est réalisée dans un ou plusieurs matériaux hypoallergéniques du type de ceux précédemment décrits. Astucieusement, l'ancre latérale (5, 5') comporte un revêtement de surface réalisée dans un ou plusieurs matériaux hypoallergéniques du type de ceux précédemment décrits. Préférentiellement, la boucle présente un orifice (51, 51') configuré pour permettre le passage d'un mors d'un clip endoscopique hémostatique. En particulier, la boucle peut présenter un orifice (51, 51') s'étendant au-delà d'un demi-cercle de 8mm de diamètre.

La partie extensible de l'organe élastique (2) peut alors être configurée pour exercer un effort de traction les ancres latérales (5, 5') lorsque sa longueur (L) est étendue au-delà de la longueur contractée (Lc).

L'ancre latérale (5, 5') peut être montée mobile par rapport à l'organe élastique (2) entre une distance latérale minimale et une distance latérale maximale. La distance latérale minimale est préférentiellement comprise entre 0 mm et 10 mm. La distance latérale maximale est préférentiellement comprise entre 10 mm et 50 mm. L'ancre latérale (5, 5') peut être montée mobile par rapport à l'organe élastique (2) par l'intermédiaire d'un fil latéral (52, 52'). Ce dernier peut être réalisé en coton, en polyester, en nylon, ou tout autre matériau convenant à l'homme du métier. Le fil latéral (52, 52') peut avoir une longueur comprise entre 10 mm et 50 mm. Dans des variantes de réalisation, l'ancre latérale (5, 5') peut être montée mobile par rapport à l'organe élastique (2) par l'intermédiaire d'une bande flexible, ou d'une tige flexible.

L'organe de réglage (6) peut être configuré pour régler la distance latérale maximale entre une distance latérale maximale initiale et une distance latérale maximale finale inférieure à la distance latérale maximale initiale.

Dans son premier mode de réalisation, l'organe de réglage (6) peut alors comprendre, pour chaque ancre latérale (5, 5'), un guide latéral (68, 68') configuré pour permettre le passage du corps (62) du collier de serrage (61). Le guide latéral (68, 68') peut être agencé sur l'ancre latérale (5, 5'). Le guide latéral peut prendre toute forme convenant à l'homme du métier.

En particulier, lorsque l'ancre latérale (5, 5') est réalisée au moyen d'un fil, le guide latéral (68, 68') peut se présenter sous la forme d'une pièce rapportée cylindrique, torique ou de toute autre forme convenant à l'homme du métier.

Également, lorsque l'ancre latérale (5, 5') est réalisée dans un matériau rigide, le guide latéral (68, 68') peut être formé d'une seule pièce avec le corps de ladite ancre latéral. Le guide latéral (68, 68') peut alors avoir la forme d'un orifice agencé sur le corps de l'ancre latérale (5, 5') ou encore sous la forme d'un cylindre, d'un anneau, ou autre, agencé en saillit sur le corps de l'ancre latérale (5, 5') vers l'intérieur ou vers l'extérieur de ladite ancre latérale.

Dans un exemple particulier de réalisation, l'orifice (51, 51') de l'ancre latéral (5, 5') fait office de guide latéral (68, 68').

Le guide latéral (68, 68') peut alternativement être agencé sur le fil latéral (52, 52'). Le guide latéral (68, 68') peut notamment être agencé à l'extrémité du fil latéral (52, 52'). Le guide latéral (68, 68') peut encore être agencé entre deux parties du fil latéral (52, 52') comme dans l'exemple de réalisation représenté sur la figure [Fig.3]. Dans ce cas, le guide latéral (68, 68') est avantageusement agencé à proximité de l'ancre latérale (5, 5'). Le guide latéral (68, 68') peut se présenter sous la forme d'une pièce rapportée cylindrique, torique ou de toute autre forme convenant à l'homme du métier. Lorsque l'ancre latérale (5, 5') est une bande, le guide latéral (68, 68') peut se présenter sous la forme d'un œillet.

Le guide latéral (68, 68') peut présenter un passage dont les dimensions sont au moins supérieures de 0.2mm, préférentiellement supérieures de 0.5mm, au diamètre, ou à l'épaisseur, du corps (62) du collier de serrage (61).

En se rapportant à l'exemple de réalisation représenté sur la figure [Fig.3], l'organe de réglage (6) peut également comprendre un second guide central (65') agencé sur l'organe élastique (2). Le second guide central (65') est du même type que le guide central (65). C'est-à-dire que le second guide central (65') procède les mêmes caractéristiques que le guide central (65) précédemment décrit, notamment en termes de forme, de matériau, d'agencement par rapport l'organe élastique (2), etc. À ce titre, le second guide central (65') est configuré pour permettre le passage du corps (62) du collier de serrage (61). Avantageusement, et comme représenté sur la figure [Fig.3], le corps (62) du collier de serrage (61) passe par le guide distal (67), puis par le guide central (65), puis par le premier guide latéral (68), puis par le guide proximal (66), puis par le deuxième guide latéral (68'), puis par le second guide central (65'). Alternativement, le corps (62) du collier de serrage (61) passe par le guide distal (67), puis par le second guide central (65'), puis le second guide latéral (66'), puis par le guide proximal (66), puis le premier guide latéral (66), puis par le guide central (65).

Dans ce premier mode de réalisation de l'organe de réglage (6), le collier de serrage (61) est alors configuré pour diminuer la distance latérale maximale lorsque la longueur de sa circonférence diminue.

Dans le deuxième mode de réalisation de l'organe de réglage (6), ce dernier peut comprendre une bobine latérale configurée pour enrouler le fil latéral (52, 52') lorsque l'effort de traction exercé sur ledit fil latéral est inférieur à un effort de rappel. L'effort de rappel est préférentiellement compris entre 0.2 N et 1.8 N. Dans un exemple de réalisation, la bobine latérale comprend un tambour configuré pour recevoir les enroulements du fil latéral (52, 52'). La bobine latérale comprend également un carter sur lequel est monté mobile en rotation le tambour. La bobine latérale comprend enfin un ressort de rappel configuré pour déclencher l'enroulement du fil latéral (52, 52') autour du tambour lorsque l'effort de traction exercé sur ledit fil latéral est inférieur à l'effort de rappel. Le carter peut être fixé sur l'organe élastique (2). Astucieusement, dans une variante de réalisation, le ressort de rappel fait office d'organe élastique (2). L'organe élastique comprend alors plusieurs éléments élastiques sous la forme d'un ressort de rappel de la bobine proximale, d'un ressort de rappel de la bobine distale, et d'un ressort de rappel de la bobine latérale. Astucieusement, le carter de la bobine proximale, le carter de la bobine distale, et le carter de la bobine latérale forment un unique carter.

L'ancre latérale (5, 5') peut être montée mobile par rapport à l'ancre distale (4) et/ou l'ancre proximale (3), entre une distance d'écartement latérale minimale et une distance d'écartement latérale maximale. La distance d'écartement latérale minimale est préférentiellement comprise entre 0 mm et 10 mm. La distance d'écartement latérale maximale est préférentiellement comprise entre 10 mm et 50 mm. En pratique, l'ensemble des modes de réalisations précédemment décrits pour l'ancre distale (4), l'ancre proximale (3) et les ancres latérales (5, 5') permettent une mobilité des ancres les unes par rapport aux autres.

L'organe de réglage (6) peut alors être configuré pour réduire la distance d'écartement maximale entre une distance d'écartement latérale maximale initiale et une distance d'écartement latérale maximale finale inférieure à la distance d'écartement latérale maximale initiale. En pratique, l'ensemble des modes de réalisations précédemment décrits pour l'organe de réglage (6) permet de réduire la distance d'écartement latérale maximale.

Un autre aspect, non revendiqué, de la présente divulgation concerne un procédé de traction d'un tissu biologique (101) au sein d'une cavité au moyen du dispositif de traction (1) objet de l'invention. Ce procédé intervient généralement dans le cadre d'une intervention de résection du tissu biologique (101) de type muqueux et présentant une tumeur (102). Le tissu biologique (101) est généralement agencé au sein de la paroi digestive (100). Au cours de cette intervention, les contours du tissu biologique (101) à réséquer sont découpés à l'aide d'un outil de dissection endoscopique. La figure [Fig.4] représente le tissu biologique (101) après découpe de ses contours (103). Le tissu biologique (101) doit ensuite être séparé du reste de la paroi digestive (100). Pour se faire, il est nécessaire d'appliquer un effort de traction sur le tissu biologique (101) de manière à l'écarter du reste de la paroi digestive (100). La sous-muqueuse (104) composer de fibre va alors s'étende et s'exposer de sorte qu'il soit possible de le disséquer au moyen d'un outil de dissection endoscopique (220). Le procédé de traction permet d'appliquer l'effort de traction sur le tissu biologique (101). Le procédé de traction comprend les étapes suivantes :
a) Fixer l'ancre proximale (3) sur une partie proximale du tissu biologique (101) au moyen d'un premier clip endoscopique hémostatique (203),
b) Fixer l'ancre distale (4) sur une partie distale du tissu biologique (101) au moyen d'un deuxième clip endoscopique hémostatique (204),
c) Fixer l'organe élastique (2) sur une paroi (105) de la cavité agencée en vis-à-vis du tissu biologique (101) au moyen d'un troisième clip endoscopique hémostatique (202) de sorte que la partie extensible de l'organe élastique (2) s'allonge au-delà de la longueur contractée (Lc) et exerce un effort de traction sur l'ancre distale (4) et sur l'ancre proximale (3).

Lorsque le tissu biologique (101) comporte une zone proximale de largeur supérieure à 3 cm, ce procédé de traction peut également comprendre l'étape suivante :
d) Fixer la deuxième ancre proximale (3') écartée de l'ancre proximale (3) fixée à l'étape a) sur la partie proximale du tissu biologique (101) au moyen d'un autre clip endoscopique hémostatique.

Lorsque le tissu biologique (101) s'étend sur plus de 8cm de long et plus d 5 cm de large, ce procédé de traction peut encore comprendre une ou plusieurs fois l'étape suivante selon le nombre d'ancres latérales que comprend le dispositif de traction :
e) Fixer une ancre latérale (5, 5') sur une partie latérale du tissu biologique (101) au moyen d'un autre clip endoscopique hémostatique.

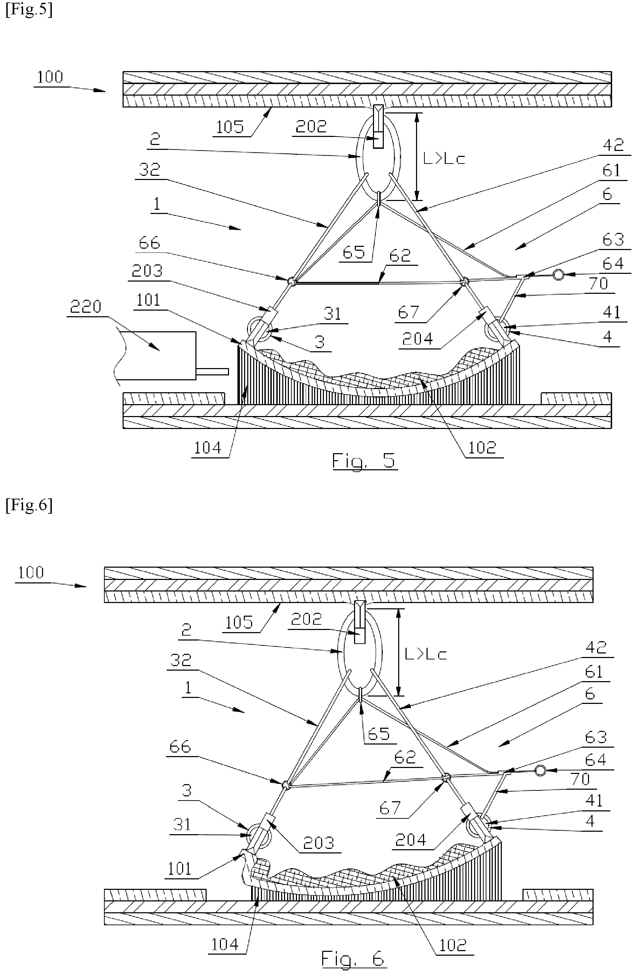

Une fois, les premières étapes du procédé de traction mises en œuvre, et comme représentée sur la figure [Fig.5], la sous-muqueuse (104) du tissue biologique (101) est suffisamment exposée. La partie proximale de la sous-muqueuse (104) est alors disséquée au moyen de l'outil de dissection (220). Néanmoins, et comme représenté sur la figure [Fig.6], une fois la partie proximale de la sous-muqueuse (104) disséquée, la partie proximale du tissu biologique (101) n'est plus contrainte par la sous-muqueuse (104) et se relâche de sorte que l'effort de traction exercé sur ledit tissu biologique n'est plus suffisant pour exposer ladite sous-muqueuse et permettre le passage de l'outil de dissection (220).

Pour résoudre ce problème, le procédé de traction comprend également l'étape suivante : d) Diminuer la distance proximale maximale au moyen de l'organe de réglage (6), lorsque l'effort de traction exercé sur l'ancre proximale (3) diminue en dessous d'un seuil (Ts).

Selon le mode de réalisation choisi pour le dispositif de traction (1), le procédé peut également comprendre indépendamment ou en combinaison les étapes suivantes :
e) Diminuer la distance distale maximale au moyen de l'organe de réglage (6), lorsque l'effort de traction exercé sur l'ancre distale (4) diminue en dessous d'un seuil (Ts),
f) Diminuer la seconde distance proximale maximale au moyen de l'organe de réglage (6), lorsque l'effort de traction exercé sur la seconde ancre proximale (3') diminue en dessous d'un seuil (Ts),
g) Diminuer la distance latérale maximale au moyen de l'organe de réglage (6), lorsque l'effort de traction exercé sur l'ancre latérale (5, 5') diminue en dessous d'un seuil (Ts),
e) Diminuer la distance d'écartement maximale au moyen de l'organe de réglage (6), lorsque l'effort de traction exercé sur l'ancre proximale (3) et/ou sur l'ancre distale (4) diminue en dessous d'un seuil (Ts),
f) Diminuer la distance d'écartement latérale maximale au moyen de l'organe de réglage (6), lorsque l'effort de traction exercé sur l'ancre proximale (3) et/ou sur l'ancre distale (4) et/ou sur l'une des ancres latérales (5, 5') diminue en dessous d'un seuil (Ts),

En pratique, le seuil (Ts) est celui à partir duquel, le passage de l'outil de dissection (220) n'est plus possible sans toucher le tissu biologique (101). Le seuil (Ts) est généralement compris entre 0.2 N et 1.8 N.

En se rapportant à la figure [Fig.7], la distance proximale maximale est diminuée par traction sur le corps (62) du collier de serrage (61) de sorte que la longueur de la circonférence dudit collier de serrage diminue. La traction est réalisée au moyen d'une pince chirurgicale endoscopique (210).

Ainsi, et comme représente sur la figure [Fig.7], une fois la distance proximale maximale diminuée, l'effort de traction exercé sur le tissu biologique (101) redevient suffisant pour exposer la sous-muqueuse (104) et permettre le passage de l'outil de dissection (220). La partie de la sous-muqueuse (104) exposée est alors disséquée. Comme précédemment, après dissection, le tissu biologique (101) se relâche et l'effort de traction devient insuffisant pour permettre de continuer la dissection. L'étape d) est alors réitéré autant de fois que nécessaire comme explicité sur la figure [Fig.8].

Un autre aspect de la présente divulgation concerne un dispositif de largage (300) du dispositif de traction (1) objet de l'invention. Le dispositif de largage (300) permet astucieusement de déposer le dispositif de traction (1) au niveau du tissu biologique (101) à tracter tout en évitant les risques d'interférence avec la paroi digestive (100) et ainsi les risques d'emmêlement des différentes ancres mobiles entre-elles.

En se rapportant aux figures [Fig.9] et [Fig. 10], le dispositif de largage (300) comprend un tube (301). Ce dernier comprend un espace interne (302) configuré pour recevoir le dispositif de traction (1). Le tube (301) peut comporter une section de profil circulaire, rectangulaire, hexagonal ou autre. La section peut avoir un diamètre, ou une longueur et une largeur, compris entre 30 mm et 70 mm. Le tube (301) peut être réalisé en matière plastique, en métal, ou tout autre matériau convenant à l'homme du métier.

Le dispositif de largage (300) comprend également un piston (303) configuré pour coulisser à l'intérieur du tube (301) de manière à pousser le dispositif de traction (1) à l'extérieur dudit tube comme représenté sur la figure [Fig.10]. Ce piston (303) peut être actionné mécaniquement ou encore par différentiel de pression. Le piston (303) peut être réalisé en matière plastique, en métal ou tout autre matériau convenant à l'homme du métier.

## Revendications

1. Dispositif de traction (1) d'un tissu biologique comprenant :
- Une ancre proximale (3),
- Une ancre distale (4),
- Un organe élastique (2) comportant au moins une partie extensible de longueur (L) et de module de Young (E) inférieur à 0,1 GPa, ledit organe élastique étant configurée pour exercer un effort de traction sur l'ancre distale (4) et sur l'ancre proximale (3) lorsque ladite longueur (L) est étendue au-delà d'une longueur contractée (Lc),
**dans lequel** l'ancre proximale (3) est montée mobile par rapport à l'organe élastique (2) entre une distance proximale minimale et une distance proximale maximale **et** ledit dispositif de traction comprend un organe de réglage (6)
configuré pour régler la distance proximale maximale entre une distance proximale maximale initiale et une distance proximale maximale finale inférieure à la distance proximale maximale initiale,
**caractérisé en ce que** l'ancre proximale (3) est montée mobile par rapport à l'ancre distale (4) entre une distance d'écartement minimale et une distance d'écartement maximale **et en ce que** l'organe de réglage (6) est configuré pour réduire la distance d'écartement maximale entre une distance d'écartement maximale initiale et une distance d'écartement maximale finale inférieure à la distance d'écartement maximale initiale..

2. Dispositif de traction selon la revendication 1 **caractérisé en ce que** l'ancre distale (4) est montée mobile par rapport à l'organe élastique (2) entre une distance distale minimale et une distance distale maximale **et en ce que** l'organe de réglage (6) est configuré pour régler la distance distale maximale entre une distance distale maximale initiale et une distance distale maximale finale inférieure à la distance distale maximale initiale

3. Dispositif de traction selon l'une des revendications précédentes **caractérisé en ce que** la partie extensible de l'organe élastique (2) a un module de Young comprise entre 0.001 GPa et 0.05 GPa.

4. Dispositif de traction selon l'une des revendications précédentes **caractérisé en ce que** l'ancre proximale (3) est montée mobile par rapport à l'organe élastique (2) par l'intermédiaire d'un fil proximal (32).

5. Dispositif de traction selon la revendication 4 **caractérisé en ce que** l'organe de réglage (6) comprend :
- Un collier de serrage (61) comportant un corps (62) et une cage (63) configurée pour permettre le passage dudit corps de manière à former une courbe fermée d'une circonférence de longueur variable,
- Un guide central (65) agencé sur l'organe élastique (2) et configuré pour permettre le passage du corps (62) du collier de serrage (61),
- Un guide proximal (66) agencé sur l'ancre proximale (3), ou sur le fil proximal (32), et configuré pour permettre le passage du corps (62) du collier de serrage (61).
- Le collier de serrage (61) étant configuré pour diminuer la distance proximale maximale lorsque la longueur de sa circonférence diminue.

6. Dispositif de traction selon l'une des revendications précédentes **caractérisé en ce que** l'ancre distale (4) est montée mobile par rapport à l'organe élastique (2) par l'intermédiaire d'un fil distal (42).

7. Dispositif de traction selon la revendication 6 en combinaison avec la revendication 5 **caractérisé en ce que** l'organe de réglage (6) comprend un guide distal (67) agencé sur l'ancre distale (4), ou sur le fil distal (42), et configuré pour permettre le passage du corps (62) du collier de serrage (61), ledit collier de serrage étant configuré pour diminuer la distance distale maximale lorsque la longueur de sa circonférence diminue.

8. Dispositif de traction selon la revendication 7 **caractérisé en ce que** l'organe de réglage (6) l'organe de réglage (6) comprend un lien de serrage (70) configuré pour maintenir la cage (63) à une distance de serrage déterminée de l'ancre distale (4).

9. Dispositif de traction selon l'une des revendications 5, 7 ou 8 **caractérisé en ce que** le collier de serrage (61) comporte un mécanisme anti-retour configuré pour empêcher le corps (62) de coulisser dans un sens dans lequel sa longueur de circonférence augmente.

## Patentansprüche

1. Traktionsvorrichtung (1) für ein biologisches Gewebe, umfassend:
- Einen proximalen Anker (3),
- Einen distalen Anker (4),
- Ein elastisches Element (2), das mindestens einen dehnbaren Abschnitt mit einer Länge (L) und einem Elastizitätsmodul (E) von weniger als 0,1 GPa aufweist, wobei das besagte elastische Element konfiguriert ist, um eine Traktionskraft auf den distalen Anker (4) und auf den proximalen Anker (3) auszuüben, wenn die besagte Länge (L) über eine zusammengezogene Länge (Lc) hinaus gedehnt wird,
**wobei** der proximale Anker (3) beweglich in Bezug auf das elastische Element (2) zwischen einem minimalen proximalen Abstand und einem maximalen proximalen Abstand montiert ist und die besagte Traktionsvorrichtung ein Einstellelement (6) umfasst,
das konfiguriert ist, um den maximalen proximalen Abstand zwischen einem anfänglichen maximalen proximalen Abstand und einem endgültigen maximalen proximalen Abstand, der geringer ist als der anfängliche maximale proximale Abstand, einzustellen,
**dadurch gekennzeichnet, dass** der proximale Anker (3) beweglich in Bezug auf den distalen Anker (4) zwischen einem minimalen Spreizabstand und einem maximalen Spreizabstand montiert ist **und dass** das Einstellelement (6) konfiguriert ist, um den maximalen Spreizabstand zwischen einem anfänglichen maximalen Spreizabstand und einem endgültigen maximalen Spreizabstand, der geringer ist als der anfängliche maximale Spreizabstand, zu verringern.

2. Traktionsvorrichtung nach Anspruch 1, **dadurch gekennzeichnet, dass** der distale Anker (4) beweglich in Bezug auf das elastische Element (2) zwischen einem minimalen distalen Abstand und einem maximalen distalen Abstand montiert ist **und dass** das Einstellelement (6) konfiguriert ist, um den maximalen distalen Abstand zwischen einem anfänglichen maximalen distalen Abstand und einem endgültigen maximalen distalen Abstand, der geringer ist als der anfängliche maximale distale Abstand, einzustellen.

3. Traktionsvorrichtung nach einem der vorhergehenden Ansprüche, **dadurch gekennzeichnet, dass** der dehnbare Abschnitt des elastischen Elements (2) einen Elastizitätsmodul zwischen 0,001 GPa und 0,05 GPa aufweist

4. Traktionsvorrichtung nach einem der vorhergehenden Ansprüche, **dadurch gekennzeichnet, dass** der proximale Anker (3) mittels eines proximalen Fadens (32) beweglich in Bezug auf das elastische Element (2) montiert ist.

5. Traktionsvorrichtung nach Anspruch 4, **dadurch gekennzeichnet, dass** das Einstellelement (6) Folgendes umfasst:
- Eine Spannschelle (61), die einen Körper (62) und einen Käfig (63) aufweist, wobei der Käfig konfiguriert ist, um den Durchtritt des besagten Körpers zu ermöglichen, so dass eine geschlossene Kurve mit einem Umfang von veränderlicher Länge gebildet wird,
- Eine zentrale Führung (65), die an dem elastischen Element (2) angeordnet und konfiguriert ist, um den Durchtritt des Körpers (62) der Spannschelle (61) zu ermöglichen,
- Eine proximale Führung (66), die an dem proximalen Anker (3) oder an dem proximalen Faden (32) angeordnet und konfiguriert ist, um den Durchtritt des Körpers (62) der Spannschelle (61) zu ermöglichen.
- Wobei die Spannschelle (61) konfiguriert ist, um den maximalen proximalen Abstand zu verkleinern, wenn sich die Länge ihres Umfangs verkleinert.

6. Traktionsvorrichtung nach einem der vorhergehenden Ansprüche, **dadurch gekennzeichnet, dass** der distale Anker (4) mittels eines distalen Fadens (42) beweglich in Bezug auf das elastische Element (2) montiert ist.

7. Traktionsvorrichtung nach Anspruch 6 in Verbindung mit Anspruch 5, **dadurch gekennzeichnet, dass** das Einstellelement (6) eine distale Führung (67) umfasst, die an dem distalen Anker (4) oder an dem distalen Faden (42) angeordnet und konfiguriert ist, um den Durchtritt des Körpers (62) der Spannschelle (61) zu ermöglichen, wobei die besagte Spannschelle konfiguriert ist, um den maximalen distalen Abstand zu verkleinern, wenn sich die Länge ihres Umfangs verkleinert.

8. Traktionsvorrichtung nach Anspruch 7, **dadurch gekennzeichnet, dass** das Einstellelement (6) ein Spannglied (70) umfasst, das konfiguriert ist, um den Käfig (63) in einem bestimmten Spannabstand von dem distalen Anker (4) zu halten.

9. Traktionsvorrichtung nach einem der Ansprüche 5, 7 oder 8, **dadurch gekennzeichnet, dass** die Spannschelle (61) einen Rücklaufsperrmechanismus aufweist, der konfiguriert ist, um zu verhindern, dass der Körper (62) in einer Richtung gleitet, in der die Länge seines Umfangs zunimmt.

## Claims

1. Traction device (1) for a biological tissue, comprising:
- A proximal anchor (3),
- A distal anchor (4),
- An elastic member (2) having at least one extensible portion of length (L) and of Young's modulus (E) lower than 0.1 GPa, said elastic member being configured to exert a traction force on the distal anchor (4) and on the proximal anchor (3) when said length (L) is extended beyond a contracted length (Lc),
**wherein** the proximal anchor (3) is movably mounted relative to the elastic member (2) between a minimum proximal distance and a maximum proximal distance and said traction device comprises an adjustment member (6) configured to adjust the maximum proximal distance between an initial maximum proximal distance and a final maximum proximal distance lower than the initial maximum proximal distance,
**characterised in that** the proximal anchor (3) is movably mounted relative to the distal anchor (4) between a minimum separation distance and a maximum separation distance **and in that** the adjustment member (6) is configured to reduce the maximum separation distance between an initial maximum separation distance and a final maximum separation distance lower than the initial maximum separation distance..

2. Traction device according to claim 1, **characterised in that** the distal anchor (4) is movably mounted relative to the elastic member (2) between a minimum distal distance and a maximum distal distance **and in that** the adjustment member (6) is configured to adjust the maximum distal distance between an initial maximum distal distance and a final maximum distal distance lower than the initial maximum distal distance.

3. Traction device according to any one of the preceding claims, **characterised in that** the extensible portion of the elastic member (2) has a Young's modulus of between 0.001 GPa and 0.05 GPa.

4. Traction device according to any one of the preceding claims, **characterised in that** the proximal anchor (3) is movably mounted relative to the elastic member (2) by means of a proximal thread (32).

5. Traction device according to claim 4, **characterised in that** the adjustment member (6) comprises:
- A clamping collar (61) having a body (62) and a cage (63) configured to allow the passage of said body so as to form a closed curve having a circumference of variable length,
- A central guide (65) arranged on the elastic member (2) and configured to allow the passage of the body (62) of the clamping collar (61),
- A proximal guide (66) arranged on the proximal anchor (3), or on the proximal thread (32), and configured to allow the passage of the body (62) of the clamping collar (61).
- The clamping collar (61) being configured to decrease the maximum proximal distance when the length of its circumference decreases.

6. Traction device according to any one of the preceding claims, **characterised in that** the distal anchor (4) is movably mounted relative to the elastic member (2) by means of a distal thread (42).

7. Traction device according to claim 6 in combination with claim 5, **characterised in that** the adjustment member (6) comprises a distal guide (67) arranged on the distal anchor (4), or on the distal thread (42), and configured to allow the passage of the body (62) of the clamping collar (61), said clamping collar being configured to decrease the maximum distal distance when the length of its circumference decreases.

8. Traction device according to claim 7, **characterised in that** the adjustment member (6) comprises a clamping link (70) configured to hold the cage (63) at a determined clamping distance from the distal anchor (4).

9. Traction device according to any one of claims 5, 7 or 8, **characterised in that** the clamping collar (61) has an anti-return mechanism configured to prevent the body (62) from sliding in a direction in which its circumference length increases.
